# EUROPEAN PATENT APPLICATION

(11) **EP 4 101 925 A1**
(43) Date of publication of application: **14.12.2022**
(21) Application number: 21750910.8
(22) Date of filing: 02.02.2021
(51) Int. Cl.: C12N 5/0783, C12N 5/0789, A61K 35/17, A61P 35/00

(54) **METHOD FOR ISOLATING AUTOLOGOUS TUMOR ANTIGEN-REACTIVE CD8 T CELL BY USING CD71, AND APPLICATION THEREOF**

(30) Priority: 03.02.2020 KR 20200012409
(71) Applicant: Samda Biotec Co., Ltd, Goyang-si, Gyeonggi-do 10408 (KR)
(72) Inventor: CHOI, Beom Kyu, Paju-si, Gyeonggi-do 10893 (KR); KIM, Seon Hee, Goyang-si, Gyeonggi-do 10450 (KR); LEE, Eun Sook, Gwacheon-si, Gyeonggi-do 13836 (KR)
(74) Representative: J A Kemp LLP
(86) International application number: PCT/KR2021/001346
(87) International publication number: WO 2021/157993

(57) **Abstract**

The present invention relates to a method for isolation of autologous cancer antigen-specific CD8 T cell by using CD71 and uses thereof. In case of isolating autologous cancer antigen-reactive CD8 T cells using the isolation method of the present invention, it is possible to provide significant anticancer effects by amplifying a large amount of CD8 T cells even with less blood since CD8 T cells responding to various cancer antigens in addition to CD8 T cells specific for autologous cancer antigen are also separated. Therefore, it can be effectively used for immunotherapy that induces temporary immunodeficiency for treatment such as adoptive cell therapy.

## Description

### [Technical field]

This application claims priority to Korean Patent Application No. 10-2020-0012409, filed on February 03, 2020, and the entire specification is incorporated by a reference to the present application.

The present invention relates to a method for isolating autologous cancer antigen-specific CD8 T cells using CD71 and uses thereof.

### [Background Art]

Adoptive T cell therapy is a method of treating cancer by isolating and mass-culturing cancer antigen-specific T cells and administering them to cancer patients. In early research, it was possible to produce CIK (cytokine-induced killer cells), LAK (lymphokine-activated killer cells), or TIL (tumor-infiltrating lymphocytes) by massively proliferating T cells in the blood or cancer tissues of cancer patients without cancer specificity. After administration to cancer patients, the safety and efficacy were evaluated. However, while safety was confirmed in most cancer patients, there was no efficacy, since the specificity for cancer of CIK, LAK, or TIL cells was low. Clinical trials of T cell therapy products to which culture methods for isolating and mass-culturing cancer antigen-specific T cells were applied were conducted sequentially, and it was reported that the anticancer effect increased when specificity for cancer cells was given. However, even using cancer cell-specific T cells, the rate of curing relapsed cancer patients was extremely low. As a method to solve the problem of low efficacy of T cell therapy, a method of administering T cell therapy after inducing temporary immunodeficiency through pre-administration of a chemical anticancer agent is used.

Anti-cancer T cell therapies that have been developed or are currently under development essentially include the concept of isolation and mass culture of cancer antigen-specific T cells. Although all T cell therapeutics have the same goal of isolating and administering cancer cell-specific T cells, the process of isolation and mass culture of T cells and the characteristics of cultured T cells are all different. Since the ratio of cancer antigen-specific T cells in blood or cancer tissue is extremely low, in general, an amplification process to increase the ratio of cancer antigen-specific T cells is usually required before isolation of cancer antigen-specific T cells. The most representative method for isolating cancer antigen-specific CD8 T cells is using MHC I/peptide multimer, but the available MHC I/peptide multimer is limited, so it cannot be applied to various patients. To overcome this limitation, an antigen-specific T cell separation process using the property of 4-1BB, which is selectively expressed only in activated T cells, has been developed (Korean Patent Registration No. 10-1503341).

The method using 4-1BB has the advantage that it can be theoretically applied to all kinds of cancer antigens. However, when transient immunodeficiency is induced for adoptive T cell therapy, about 10¹¹ or more lymphocytes are removed, and this method only provides one cancer antigen-specific CD8 T cell at the scale of 10⁹ cells. This may increase the possibility of immune evasion of cancer cells or the possibility of side effects due to immunodeficiency, so a solution thereto is required.

### [Disclosure]

### [Technical Problem]

Accordingly, the present inventors made diligent efforts to provide a method for isolating CD8 T cells that respond to autologous cancer antigens from a small amount of blood and amplifying them to a significant level that can be applied to cell therapy within a short period of time. When peripheral blood mononuclear cells (PBMC) were stimulated by autologous cancer antigen, CD8 T cells that can respond to various cancer antigens in addition to autologous cancer antigen-specific CD8 T cells proliferated together, and when they were isolated using CD71, a significant anticancer effect was provided. It was confirmed that CD8 T cells showing a significant anticancer effect could be isolated and proliferated, and the present invention was completed.

Accordingly, it is an object of the present invention to provide a method for isolating autologous cancer antigen-specific CD8 T cells using CD71 and uses thereof.

### [Technical solution]

The present invention provides a method for isolating an autologous cancer antigen-specific CD8 T cell, comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a); and
d)isolating CD71+CD8+ T cells from the cultured PBMCs of step c).

According to a preferred embodiment of the present invention, the cancer of step a) is at least one selected from the group consisting of gastric cancer, lung cancer, pancreatic cancer, melanoma, brain tumor, leukemia, ovarian cancer and sarcoma.

According to a preferred embodiment of the present invention, the autologous cancer antigen of step a) is at least one selected from the group consisting of hTERT, WT-1, NY-ESO-1 and MAGE-3.

According to a preferred embodiment of the present invention, the selection in step b) is to select an autologous cancer antigen-derived peptide that highly expresses 4-1BB-expressing autologous cancer antigen-specific CD8 T cells.

According to a preferred embodiment of the present invention, the blood of the cancer patient in step c) is 10 to 100 ml.

According to a preferred embodiment of the present invention, the culturing in step c) is performed for 12 to 17 days.

The present invention also provides a pharmaceutical composition for preventing or treating cancer comprising the isolated autologous cancer antigen-specific CD8 T cells.

The present invention also provides a method for improving or treating cancer, comprising administering to a cancer patient a composition comprising the isolated autologous cancer antigen-specific CD8 T cells.

The present invention also provides a method for producing a composition for anticancer cell therapy comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a);
d) isolating CD71+CD8+ T cells from the cultured PBMC of step c); and
e) amplifying the CD71+CD8+ T cells isolated in step d).

According to a preferred embodiment of the present invention, the separation of step d) is performed when the ratio of CD71+CD8+ T cells in PBMC is 1 to 25%.
present invention, the amplification of step e) is carried out for 12 to 17 days to amplify 10⁹ to 10¹¹ CD71+CD8+ T cells.

The 'autologous cancer antigen-specific CD8 T cell' of the present invention refers to a CD8 T cell capable of responding to various cancer antigens, including autologous cancer antigens.

The 'Bystander activation' of the present invention means that T cells specific for another antigen are activated during an immune response to a specific antigen.

The 'cell therapy agent' of the present invention refers to a cell and tissue manufactured through isolation, culture, or special manipulation from an individual, and refers to a drug used for the purpose of treatment, diagnosis, or prevention. It refers to a drug in which allogeneic or xenogeneic cells are used for the purpose of treatment, diagnosis, or prevention of diseases through a series of actions such as proliferation and selection in vitro or changing the biological properties of cells by other methods.

As described above, the CD8 T cell amplification method used in the existing cell therapy lacks the amount of amplified CD8 T cells to recover the immune deficiency induced for cell therapy, and reacts specifically to only one cancer antigen. In many cases, it was insufficient to obtain a significant anticancer effect.

In case of using the autologous cancer antigen-specific CD8 T cell isolation method of the present invention, >10¹⁰ anticancer CD8 T cells can be cultured from 50 to 100 ml of cancer patient blood for about 25 days. Thus, even if grade 3-4 level of lymphopenia occurs through the administration of anticancer drugs to enhance the anticancer effect of cells and a large amount of lymphocytes are removed, it is possible to produce administrable anticancer CD8 T cells at a level that can compensate for this.

hTERT and WT1 are representative autologous cancer antigens, and they exist in extremely low proportions in the body of healthy people, making it difficult to proliferate. In cancer patients, CD8 T cell responses to various cancer antigens including hTERT are activated, which makes it possible to proliferate and separate autologous cancer antigen-specific CD8 T cells, such as hTERT, differently from healthy people. However, the process of culturing anticancer CD8 T cells using peptides derived from autologous cancer antigens is possible only when these cells are present in a sufficiently high proportion in the body of a cancer patient.

The present invention provides not only hTERT or WT1 peptide-specific CD8 T cells used for CD8 T cell proliferation through CD71+CD8 T cell isolation, but also continuously raised in response to the proliferation of cancer cells. Some of the cells can proliferate and separate through bystander activation. Therefore, the final cultured anticancer CD8 T cells can induce a very good anticancer effect because they contain other cancer antigen-specific CD8 T cells self-proliferated by cancer patients along with hTERT or WT1.

Accordingly, the present invention provides a method for isolating an autologous cancer antigen-specific CD8 T cell, comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a); and
d) isolating CD71+CD8+ T cells from the cultured PBMCs of step c).

The present invention also provides an autologous cancer antigen-specific CD8 T cell isolated by the method of the present invention.

According to a preferred embodiment of the present invention, the cancer of step
a) may be any one or more selected from the group consisting of gastric cancer, lung cancer, pancreatic cancer, melanoma, brain tumor, leukemia, ovarian cancer and sarcoma.

According to a preferred embodiment of the present invention, the autologous cancer antigen in step a) may be at least one selected from the group consisting of hTERT, WT-1, NY-ESO-1 and MAGE-3. More preferably, it may be any one or more selected from the group consisting of hTERT and WT-1.

According to a preferred embodiment of the present invention, the autologous cancer antigen-derived peptide may be a peptide derived from any one or more autologous cancer antigens selected from the group consisting of hTERT, WT-1, NY-ESO-1 and MAGE-3. More preferably, it may be a peptide derived from any one or more autologous cancer antigens selected from the group consisting of hTERT or WT-1, and more preferably a peptide of SEQ ID NO: 2 to SEQ ID NO: 59.

According to a preferred embodiment of the present invention, the selection of step b) may be selection of autologous cancer antigen-derived peptides that highly express 4-1BB-expressing autologous cancer antigen-specific CD8 T cells. Specifically, autologous cancer antigen-derived peptides may be selected in which the ratio of 4-1BB-expressing CD8 T cells among all CD8 T cells is 10% or more.

According to a preferred embodiment of the present invention, the blood of the cancer patient in step c) may be 10 to 100 ml. Preferably, the blood of a cancer patient is 20 to 80 ml, and more preferably 30 to 50 ml.

According to a preferred embodiment of present invention, the culturing in step c) may be performed for 12 to 17 days. Preferably, it may be carried out for 13 to 16 days, and more preferably, it may be carried out for 14 to 15 days.

The present invention may also provide a pharmaceutical composition for preventing or treating cancer comprising the isolated autologous cancer antigen-specific CD8 T cells.

The present invention may be in various parenteral formulations. Formulations for parenteral administration may include sterile aqueous solutions, non-aqueous solutions, suspensions, emulsions, lyophilized formulations, or suppositories. For non-aqueous solvents and suspensions, Propylene glycol, polyethylene glycol, vegetable oils such as olive oil, and injectable esters such as ethyl oleate may be used. As the base of the suppository, witepsol, macrogol, tween 61, cacao butter, laurin fat, glycerol, gelatin, etc. can be used.

The pharmaceutical composition of the present invention may be administered parenterally. When administered parenterally, it can be formulated according to methods known in the art in the form of injections for skin external, intraperitoneal, rectal, intravenous, intramuscular, subcutaneous, intrauterine dural or intracerebrovascular injection.

In case of the above injection, it must be sterilized and protected from contamination of microorganisms such as bacteria and fungi. For injection, examples of suitable carriers may include, but are not limited to, water, ethanol, polyols (e.g., glycerol, propylene glycol and liquid polyethylene glycol, etc.), mixtures thereof and/or a solvent or dispersion medium containing vegetable oil. More preferably, suitable carriers may be used such as, Hanks' solution, Ringer's solution, phosphate buffered saline (PBS) with triethanolamine or isotonic solutions such as sterile water for injection, 10% ethanol, 40% propylene glycol and 5% dextrose. etc. In order to protect the injection from microbial contamination, it may further include various antibacterial and antifungal agents such as parabens, chlorobutanol, phenol, sorbic acid, thimerosal, and the like. In addition, in most cases, the injection may further contain an isotonic agent such as sugar or sodium chloride.

The pharmaceutical composition of the present invention is administered in a pharmaceutically effective amount. A pharmaceutically effective amount means an amount sufficient to treat a disease with a reasonable benefit/risk ratio applicable to medical treatment, and the effective dose level is the patient's disease type, severity, drug activity, drug sensitivity, and administration time., administration route and excretion rate, duration of treatment, factors including concomitant drugs, and other factors well known in the medical field. The pharmaceutical composition of the present invention may be administered as an individual therapeutic agent or may be administered in combination with other therapeutic agents, may be administered sequentially or simultaneously with conventional therapeutic agents, and may be administered single or multiple. That is, the total effective amount of the pharmaceutical composition of the present invention may be administered to a patient as a single dose, and may be administered by a fractionated treatment protocol in which multiple doses are administered for a long period of time. In consideration of all of the above factors, it is important to administer an amount that can obtain the maximum effect with a minimum amount without side effects, which can be easily determined by a person skilled in the art.

The present invention can be used alone or in combination with methods using surgery, radiation therapy, hormone therapy, chemotherapy, and biological response modifiers.

The present invention may also provide a method for improving or treating cancer, comprising administering to a cancer patient a composition comprising the isolated autologous cancer antigen-specific CD8 T cells.

The present invention can be administered in an amount sufficient to treat a disease at a reasonable benefit/risk ratio applicable to medical treatment, and the patient's disease type, severity, drug activity, sensitivity to drug, administration time, administration route and excretion rates, duration of treatment, factors including concomitant drugs, and other factors well known in the medical field. The composition of the present invention may be administered as an individual therapeutic agent or may be administered in combination with other therapeutic agents, may be administered sequentially or simultaneously with conventional therapeutic agents, and may be administered single or multiple. That is, the total effective amount of the composition of the present invention may be administered to a patient as a single dose, and may be administered by a fractionated treatment protocol in which multiple doses are administered for a long period of time. In consideration of all of the above factors, it is important to administer an amount that can obtain the maximum effect with a minimum amount without side effects, which can be easily determined by a person skilled in the art.

The present invention also provides a method for producing a composition for anticancer cell therapy comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a);
d) isolating CD71+CD8+ T cells from the cultured PBMC of step c); and
e) amplifying the CD71+CD8+ T cells isolated in step d).

The present invention also provides a composition for anticancer cell therapy prepared by the method of the present invention.

According to a preferred embodiment of the present invention, the separation of step d) may be performed when the ratio of CD71+CD8+ T cells in PBMC is 1 to 25%, and preferably, the ratio is 4 to 25%. and more preferably 10 to 25%. When the ratio of CD71+CD8+ T cells in PBMC exceeds 10 %, a recovery rate similar to the purity of CD71+CD8+ T cells isolated from PBMC can be stably obtained (Example 6).

According to a preferred embodiment of the present invention, the amplification of step e) is carried out for 12 to 17 days to amplify 10⁹ to 10¹¹ CD71+CD8+ T cells. The amplification of step e) may be preferably performed for 13 to 16 days, and more preferably, may be performed for 14 to 15 days. The CD71+CD8+ T cells may be amplified to preferably 10⁹ to 10¹⁰.

### [Advantageous Effects]

In case of isolating autologous cancer antigen-reactive CD8 T cells using the isolation method of the present invention, it is possible to provide significant anticancer effects by amplifying a large amount of CD8 T cells even with less blood since CD8 T cells responding to various cancer antigens in addition to CD8 T cells specific for autologous cancer antigen are also separated. Therefore, it can be effectively used for immunotherapy that induces temporary immunodeficiency for treatment such as adoptive cell therapy.

### [Description of Drawings]

Figure 1 shows 4-1BB and CD71 expression in proliferating Melan-A specific and bystander activated CD8+ T cells.

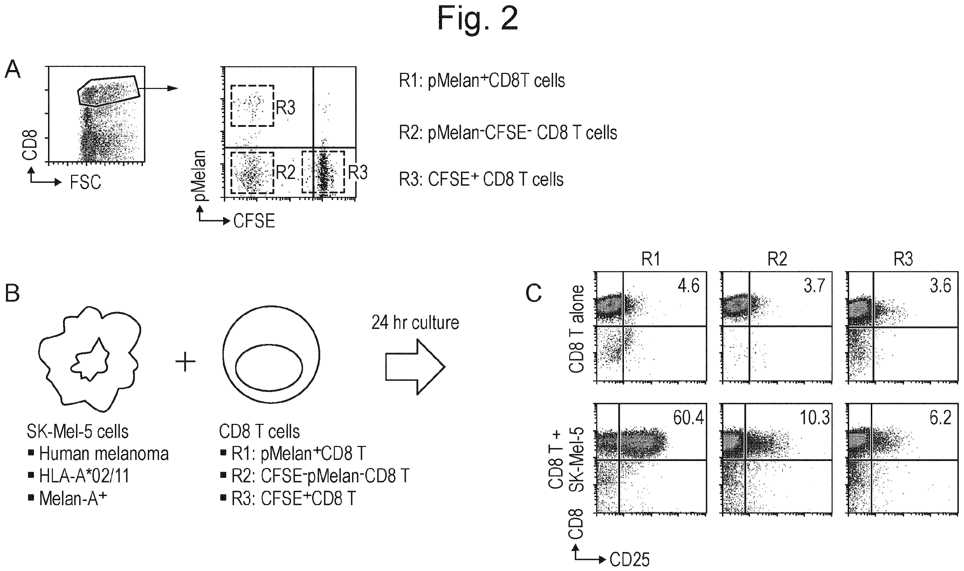

Figure 2 shows that CD8+ T cells proliferated by bystander activation in the presence of Melan-A peptide include human-tumor-reactive CD8+T cells. (A): Cultured PBMCs were stained with PE-conjugated Melan-A peptide/MHC class I multimer (pMelan) and anti-CD8-PE-Cy5. The gated CD8+ cells were plotted as CFSE versus pMelan and the results of flow cytometry analysis of the indicated CD8+ T cells were shown. (B~C): Three different CD8+ T cells of pMelan+CD8 T(R1), CFSE-pMelan-CD8 T(R2) and CFSE+ CD8 T(R3) cells were amplified using rapid expansion method to anti-CD8-PE-The results of flow cytometry analysis after staining with Cy5 and anti-CD25-PE were shown.
Figure 3 shows the killing of Melan-A+ and Melan-A-melanoma cells with expanded CD71+ CD8+ T cells.
Figure 4 shows the expression of 4-1BB and CD71 in CD8+ T cells activated by hTERT peptide. The red dotted box indicated PBMCs containing a higher percentage of 4-1BB+CD8+ T cells, and the PBMCs were finally used for sorting CD71+CD8+ T cells.
Figure 5 shows the expansion of sorted CD71+ CD8+ T cells and their phenotypes. Figure 5 (A) showed the results of pooling selected PBMCs and flow cytometry analysis of CD71+CD8+ T cells. (B): Sorted CD71+ CD8+ T cells were proliferated using the rapid expansion method, and 5×10⁵ cells became >1×10⁹ cells within 13 days. (C): Expanded CD8+ T cells were stained with anti-CD8-PE-Cy5 and PE-conjugated anti-CD45RA, anti-CD45RO, anti-CD57 or anti-PD-1. Figure 5 (D) showed the result of confirming the viability of the expanded CD8 T cells by staining with Anexin V-FITC and PI.
Figure 6 shows that CD71+ CD8+ T cells include WT1 peptide-specific CD8+T cells.
Figure 7 (A): Melan-A-positive or negative CD8+ T cells were isolated from PBMCs of healthy volunteers bearing the HLA-A^{∗}0201 allele using Melan-A CTL peptide. (B): Expanded pMelan+ and pMelan-CD8+ T cells were mixed at different ratios, and pMelan-CD8+ T cells were sorted and counted from the 5×10⁶ cell mixture using an automated cell sorter (Tyto) and analyzed by flow cytometry. (C) shows the results of calculating the purity and recovery rate of pMelan+CD8+ T cells from the results of (B).

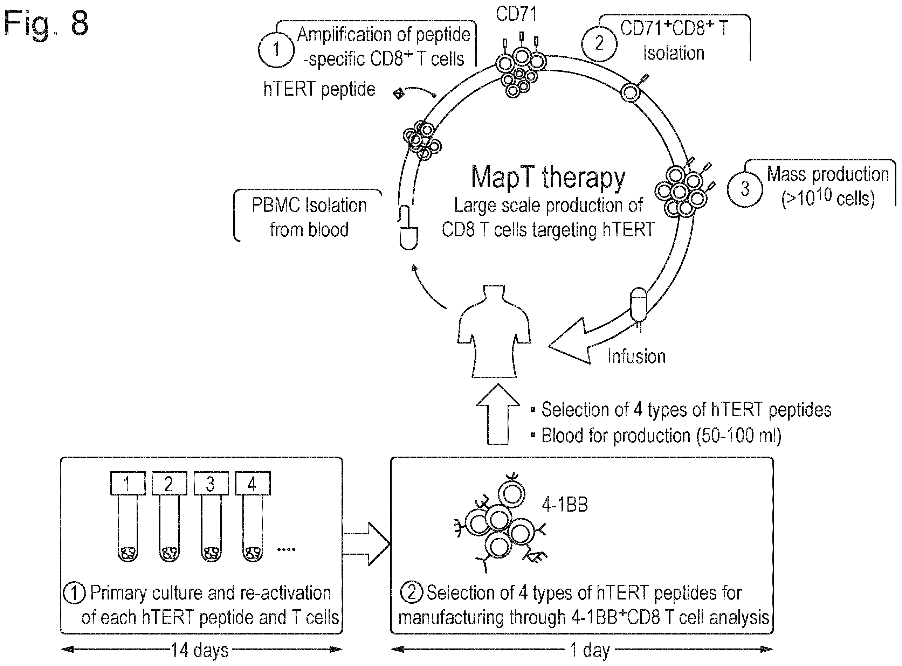

Figure 8 shows a workflow for producing tumor-reactive CD8 T cells, including hTERT-specific CD8 T cells, from the blood of a cancer patient.

### [Mode of the Invention]

### [Example 1]

### Determination of CD71 Expression in CD8 T Cells

The purpose of this example was to prove that CD8 T cells proliferating by CD8 T cell proliferation peptide express CD71 using the autoantigen Melan-A specific CD8 T cells. All blood samples had HLA-A^{∗}02 allele and were provided after written consent from healthy volunteers with HLA-A0201/Melan-A₂₆₋₃₅ (SEQ ID NO: 1: ELAGIGILTV) multimer (pentamer) positive CD8 T cells.

Specifically, Melan-A₂₆₋₃₅ specific CD8 T cells were induced to proliferate using Melan-A₂₆₋₃₅ (SEQ ID NO: 1: ELAGIGILTV) peptide, which is a cytotoxic T lymphocyte (CTL) epitope of Melan-A antigen. The blood of the healthy volunteers (Donor #1 and #2) was collected in 10 ml BD Vacutainer Heparin Tube (BD Bioscience). To isolate peripheral blood mononuclear cells (PBMC) from the blood collected, 5 ml Ficoll-Paque (GE Healthcare) was added to a 15 ml conical tube, and then 10 ml of the collected blood was slowly dispensed on the upper layer of the Ficoll solution. After centrifugation at 800×g, no brake, for 20 minutes, the plasma of the top layer was collected, filtered with a 0.2 µM syringe filter, and used as autologous plasma. PBMCs were collected, washed twice with RPMI1640 medium, and used for culture.

The isolated PBMCs were stained with 10 µM CFSE (CellTrace CFSE Cell Proliferation kit, Thermo Fisher) for 5 minutes. CFSE-labeled PBMCs were suspended in RPMI1640 medium containing 3% autologous plasma at a concentration of 1×10⁶ cells/mL, and each 1 mL was dispensed into a 14 mL round tube (BD Bioscience). The Melan-A₂₆₋₃₅ peptide was added at a concentration of 2 µg/ml to each tube at a concentration of 2 µg/ml, and then incubated in a CO₂ incubator. On the second day of culture, RPMI1640 medium containing 100 IU/ml IL-2 and 3% autologous plasma was added to each tube by 1 ml. On the 7th, 9th, 11th or 13th day of culture, 1 ml of medium was removed from each tube, and 1 ml of fresh RPMI1640 medium containing 100 IU/ml IL-2 and 3% autologous plasma was added and cultured for 14 days. On the 7th, 9th, 11th or 14th day of culture, PBMCs from the tube were collected and washed once with RPMI1640 medium, and then were stained by pMelan-PE, anti-4-1BB-PE, or anti-CD71-PE antibody with anti-CD8-PE-Cy5 and flow cytometry was performed with a FACSCalibur (BD Bioscience).

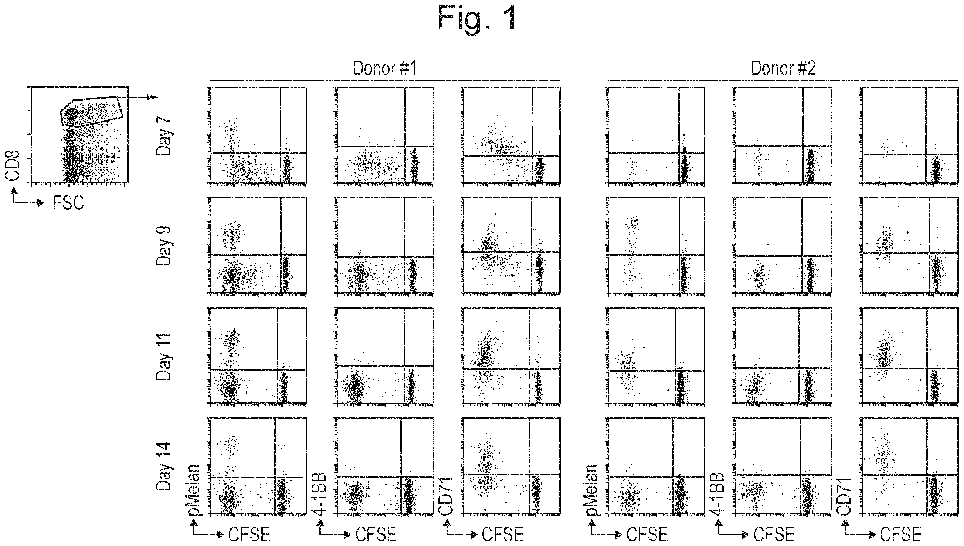

As a result, as shown in [Fig. 1], on the 7th day of culture, most of the pMelan+CD8 T cells had more than 9 cell divisions and became a CFSE-negative state, while at the same time CFSE-pMelan-CD8 T cells were also found in high proportion by bystander activation. Donor #1 was a volunteer with a high ratio of pMelan⁺CD8 T cells, and the proliferation rate of CD8 T cells was also high. Donor #2 was a volunteer with a low ratio of pMelan⁺CD8 T cells and the proliferation rate of CD8 T cells was low. Although the ratio of pMelan⁺CD8 T cells steadily increased until day 11 of culture, it was confirmed that it partially decreased on day 14 of culture (left of FIG. 1). On day 7 of culture, expression of 4-1BB was detected in some of the proliferating CFSE⁻CD8 T cells, but not after day 9 (middle of FIG. 1). As the CD8 T cell proliferation progressed and the CFSE fluorescence value decreased, the expression of CD71 was found to increase, and it was confirmed that most of the CD8 T cells proliferated 9-10 times or more on the 14th day of culture expressed CD71 (right of Fig. 1).

The above results were judged to mean that most of the CD8 T cells proliferated specifically and non-specifically for Melan-A peptides expressed CD71.

### [Example 2]

### CD8 T cell activation by Melan-A-derived CTL peptide

In this example, it was attempted to determine whether the CD8 T cells proliferated through the bystander activation confirmed in Example 1 can recognize melanoma.

Specifically, all of the cultured PBMCs of <Example 1> were collected, stained with pMelan-PE and anti-CD8-PE-Cy5, and then pMelan+CD8 T (R1), CFSE-pMelan-CD8 T(R2) and CFSE+CD8 T(R3) cells were isolated, respectively among gated CD8 T cells using FACSAria (BD Bioscience). The isolated three types of CD8 T cells were cultured for 14 days using the rapid expansion method and mass-proliferated to >10⁹ level.

After mixing 5×10⁵ finally isolated CD8 T cells, 1×10⁸ irradiated allogeneic PBMCs, 40 ng/mL anti-human CD3 mAb, 1,000 IU/mL rhIL-2, and 3% autologous plasma in 50 mL ALyS505N (CSTI, Japan) medium and the mixture was injected into a 1L culture bag (Nipro, Japan), and cultured for 14 days. During culture, 50 ml of the above medium on the 4th day of culture, 100 ml of the medium on the 7th day of culture, 300 ml of the medium on the 9th day of culture, and 500 ml of the medium on the 11th day of culture were added, respectively. After dispensing 2×10⁶ final cultured CD8 T cells and 2×10⁵ human melanoma cell line SK-Mel-5 (10:1) into a 24 well culture plate, it was cultured for 24 hours in the presence of 100 IU/ml rhIL-2 and 3% autologous plasma, and as a negative control, only CD8 T cells were cultured without SK-Mel-5. After incubation, cells were corrected, stained with anti-CD8-PE-Cy5 and anti-CD25-PE antibodies, and analyzed with FACSCalibur (BD Bioscience) to determine the proportion of CD8 T cells activated by SK-Mel-5.

As a result, as shown in [FIG. 2], it was confirmed that some pMelan⁻CD8 T cells proliferated together through bystander activation along with the proliferation of pMelan⁺CD8 T cells (FIG. 2A). When only pMelan⁺CD8 T (R1), CFSE⁻pMelan-CD8 T (R2) and CFSE⁺CD8 T (R3) cells were cultured for 24 hours, the expression of CD25 as an activity marker was maintained at a low level of 3-5%. However, when HLA-A^{∗}02⁺SK-Mel-5 cells and CD8 T cells were co-cultured for one day, pMelan⁺CD8 T cells were rapidly activated and the proportion of CD25-expressing cells was found to be 60%. Therefore, it was judged that HLA-A^{∗}02⁺SK-Mel-5 cells can induce CD8 T cell activity by normally loading Melan-A peptides on MHC class I and arranging them on the cell surface. At the same time, among CD8 T cells corresponding to R2 and R3, CD25 expression was increased after co-culture with SK-Mel-5. It was confirmed that the ratio of cells responding to SK-Mel-5 was higher in CFSE⁻pMelan-CD8 T cells than in CFSE⁺CD8 T cells, which are non-dividing CD8 T cells (FIG. 2C).

The above result was judged to mean that the CD8 T cells proliferated by the bystander activation effect of the Melan-A peptide contained cancer antigen-specific CD8 T cells at a high ratio.

### [Example 3]

### Confirmation of cancer cell removal ability of CD71⁺CD8⁺T cells

Melan-A CTL peptide was added to PBMCs of healthy volunteers bearing HLA-A^{∗}0201-restricted Melan-A-specific CD8+T cells and they were cultured for 14 days. After separation and mass culture of CD71+CD8+ and 4-1BB+CD8+ T cells, the cells were co-cultured with the Melan-A-positive SK-Mel-5 cell line and the Melan-A-negative A375 cell line to compare their ability to remove cancer cells.

Specifically, from the PBMCs cultured in <Example 1>, on the 14th day of culture, CD71+CD8+ T cells were separated using a cell sorter (FACSAria, BD Bioscience). In the case of 4-1BB+CD8+ T cells, the cells were harvested on the 14th day of culture, washed twice with RPMI1640 medium, and then suspended in CTL media at 2×10⁶ cells/ml and aliquoted in a 12 well culture plate, the same Melan-A CTL peptide was added at a concentration of 5 µg/ml and re-stimulated for one day to isolate 4-1BB+CD8+ T cells. The isolated 4-1BB+CD8+ T and CD71+CD8+ T cells were mass-cultured for 14 days using the rapid expansion method.

Melan-A-positive SK-Mel-5 cell line or Melan-A-negative A375 cell line having HLA-A^{∗}0201 allele was stained with 10 µM CFSE for 5 minutes for fluorescent labeling, and then washed with RPMI1640 medium to prepare target cells. 1×10⁵ fluorescently labeled SK-Mel-5 or A375 cell lines and the final cultured 4-1BB+CD8+ T and CD71+CD8+ T cells were mixed at a ratio of 0, 0.1, 0.2, 0.5, 1 or 5 times., CTL medium containing 100 IU/ml rhIL-2 and 3% autologous plasma for 6 hours at 37°C, and incubated in an 5% CO₂ incubator. After completion of culture, cells were stained with annexin-PE and flow cytometry was performed to analyze the percentage of apoptotic cells.

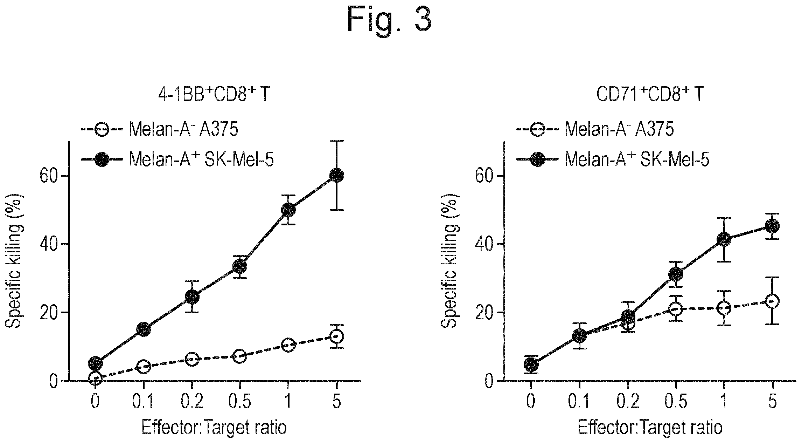

As a result, as shown in [Fig. 3], 4-1BB+CD8+ T cells containing only Melan-A-specific CD8 T cells selectively removed only Melan-A+ SK-Mel-5 cell lines, and in the case of CD71+CD8+ T cells containing bystander-activated CD8 T cells along with Melan-A-specific CD8 T cells, it was confirmed that all of the Melan-A+ SK-Mel-5 cell lines as well as the Melan-A-A375 cell lines were removed.

### [Example 4]

### CD8 T cell activation by hTERT-derived CTL peptide

The purpose of this example was to investigate whether hTERT-specific and bystander activated CD8 T cells can be proliferated in PBMCs of cancer patients using hTERT-derived CTL peptides.

**[Table 1]**

| title | sequence | sequence list |
|---|---|---|
| hTERT 1 | AAFRALVAQCL | SEQ ID NO: 2 |
| hTERT 2 | CLKELVARV | SEQ ID NO: 3 |
| hTERT 3 | LAFGFALL | SEQ ID NO: 4 |
| hTERT 4 | VGDDVLVH | SEQ ID NO: 5 |
| hTERT 5 | FVLVAPSCA | SEQ ID NO: 6 |
| hTERT 6 | GAATQARP | SEQ ID NO: 7 |
| hTERT 7 | SGTRHSH | SEQ ID NO: 8 |
| hTERT 8 | KEQLRPSFLLSSLRPSL | SEQ ID NO: 9 |
| hTERT 9 | PLFLELL | SEQ ID NO: 10 |
| hTERT 10 | AAVTPAA | SEQ ID NO: 11 |
| hTERT 11 | QSIGIRQ | SEQ ID NO: 12 |
| hTERT 12 | IVNMDYV | SEQ ID NO: 13 |
| hTERT 13 | RPGLLGASV | SEQ ID NO: 14 |
| hTERT 14 | TLTDLQP | SEQ ID NO: 15 |
| hTERT 15 | LLCSLCYG | SEQ ID NO: 16 |
| hTERT 16 | LVRGVPEYGCVVNLR | SEQ ID NO: 17 |
| hTERT 17 | YSSYARTSIRASL | SEQ ID NO: 18 |
| hTERT 18 | IYKILLLQAY | SEQ ID NO: 19 |
| hTERT 19 | LGAKGAA | SEQ ID NO: 20 |
| hTERT 20 | YVPLGSL | SEQ ID NO: 21 |
| hTERT 21 | QTQLSRKLP | SEQ ID NO: 22 |
| hTERT 22 | ALEAAANP AL | SEQ ID NO: 23 |
| hTERT 23 | ILAKFLHWL | SEQ ID NO: 24 |
| hTERT 24 | RLVDDFLLV | SEQ ID NO: 25 |
| hTERT 25 | EARPALLTSRLRFIPK | SEQ ID NO: 26 |
| hTERT 26 | RLFFYRKSV | SEQ ID NO: 27 |
| hTERT 27 | YLFFYRKSV | SEQ ID NO: 28 |
| hTERT 28 | DLQVNSLQTV | SEQ ID NO: 29 |
| hTERT 29 | YLQVNSLQTV | SEQ ID NO: 30 |
| hTERT 30 | GLLGASVLGL | SEQ ID NO: 31 |
| hTERT 31 | ALL TSRLRFI | SEQ ID NO: 32 |
| hTERT 32 | RLT SRVKAL | SEQ ID NO: 33 |
| hTERT 33 | TYVPLGSL | SEQ ID NO: 34 |
| hTERT 34 | CYGDMENKL | SEQ ID NO: 35 |
| hTERT 35 | AYQVCGPP | SEQ ID NO: 36 |
| hTERT 36 | VYGFVRACL | SEQ ID NO: 37 |
| hTERT 37 | VYAETKHFL | SEQ ID NO: 38 |
| hTERT 38 | DYVVGARTF | SEQ ID NO: 39 |

Specifically, PBMCs isolated in the same manner as in <Example 1> from 30 ml of blood of a relapsed lung cancer patient who did not respond to standard treatment were cultured with CTL peptides derived from 38 types of hTERT in [Table 1] for 14 days. On the 14th day of culture, they were re-stimulated with the same peptide for one day, and on the 15th day of culture, some of the PBMCs in each tube were stained with anti-4-1BB-PE or anti-CD71-PE and anti-CD8-PE-Cy5. Through flow cytometry, hTERT peptides containing 4-1BB⁺CD8⁺T cells and at the same time showing a high ratio of CD71+CD8+T cells were selected. After pooling the PBMCs in the selected tube, CD71⁺CD8⁺T cells were isolated using FACSAria, and the isolated CD8 T cells were mass-cultured for 14 days using the rapid expansion method, and the final cultured CD8 T cells were analyzed for phenotype using ani-CD45RA, anti-CD45RO, anti-CD57 or anti-PD-1 antibody.

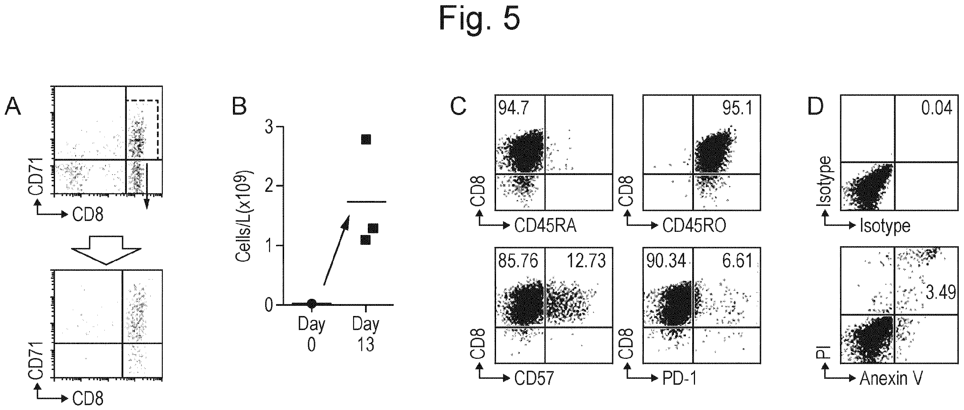

In addition, separately from this, PBMCs from 8 finally selected tubes were pooled, and CD71+CD8+T cells were isolated using FACSAria and stained with anexin V-FITC and PI to determine the cell viability (FIG. 5A).

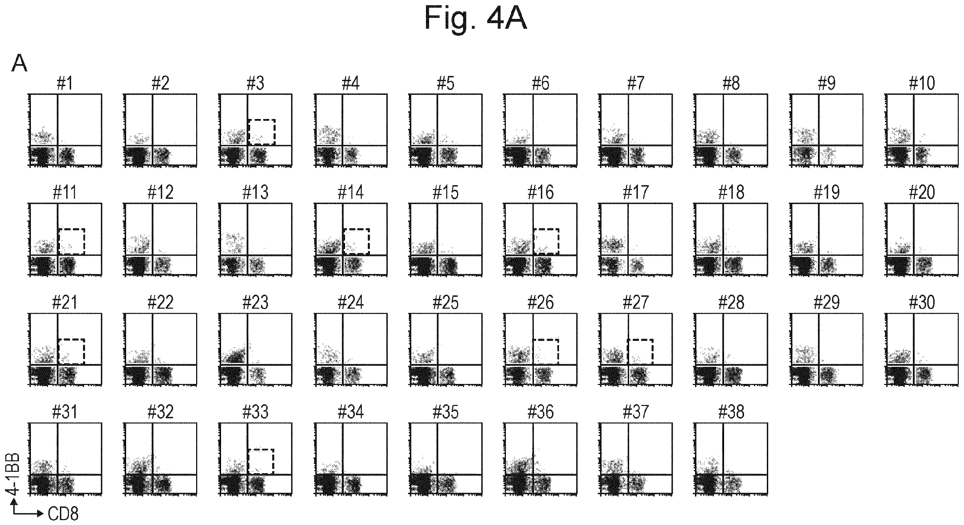

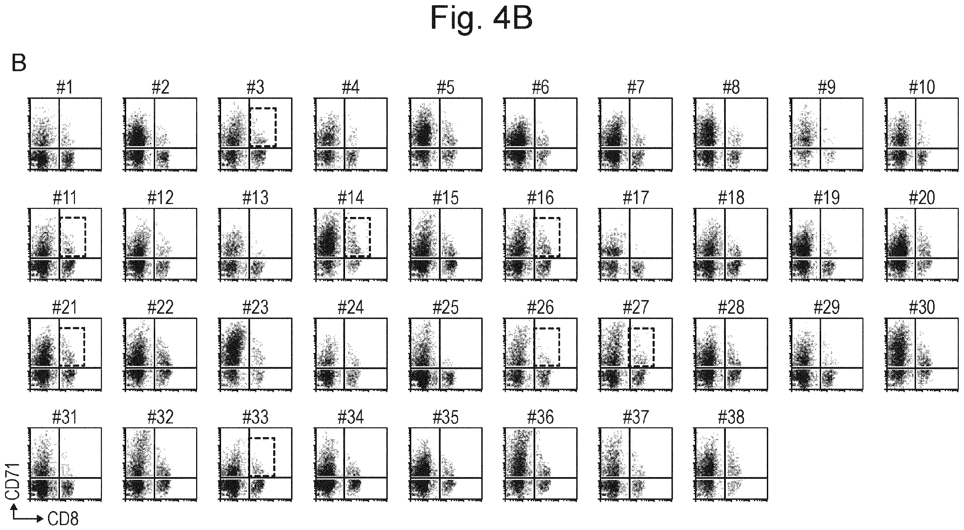

As a result, as shown in [Fig. 4], it was confirmed that 4-1BB+CD8 T cells were increased by various hTERT peptide stimuli.

It was confirmed that 4-1BB+CD8+ T cells were CD8 T cells that were specifically activated by the added peptide. PBMCs (red dotted line boxes) in the selected tube expressed not only 4-1BB but also CD71 at a higher ratio, so it was determined that they contained the added hTERT peptide-specific and bystander-activated CD8 T cells.

In addition, as shown in B of FIG. 5, when isolated CD71+CD8+T cells were mass-proliferated for 14 days using the rapid expansion method, 5×10⁵ cells became 1~3×10⁹ cells within 14 days. It was confirmed that the final proliferated CD8 T cells were CD45RA⁻CD45RO⁺ memory type cells, the proportion of senescent cells expressing CD57 was within 15%, and the proportion of exhausted CD8 T cells expressing PD-1 was within 10% (FIG. 5C). When cell viability was measured through Anexin V and PI staining, it was confirmed that the percentage of apoptotic cells was within 5% (FIG. 5D).

The above results showed that when hTERT-specific CD8 T cells were present in PBMCs in the blood of cancer patients, bystander-activated CD8 T cells increased along with their proliferation, and it was judged to mean that it was possible to isolate and mass-culture bystander-activated CD8 T cells by hTERT peptide along with hTERT-specific CD8 T cells using CD71.

### [Example 5]

### CD8 T cell activation by WT1-derived CTL peptide

Similar to the case of hTERT, the purpose of this example was to investigate whether WT1-specific and bystander-activated CD8 T cells can be proliferated from PBMCs of cancer patients using CTL peptides derived from WT1 cancer antigen.

**[Table 2]**

| title | sequence | sequence list |
|---|---|---|
| WT1 1 | ALLPAVPSL | SEQ ID NO: 40 |
| WT1 2 | DLNALLP A V | SEQ ID NO: 41 |
| WT1 3 | SLGEQQYSV | SEQ ID NO: 42 |
| WT1 4 | RMFPNAPYL | SEQ ID NO: 43 |
| WT1 5 | GVFRGIQDV | SEQ ID NO: 44 |
| WT1 6 | CMTWNQMNL | SEQ ID NO: 45 |
| WT1 7 | SGQFTGTAGA | SEQ ID NO: 46 |
| WT1 8 | VLDFAPPGA | SEQ ID NO: 47 |
| WT1 9 | A YPGCNKRYF | SEQ ID NO: 48 |
| WT1 10 | QYRIHTHGVF | SEQ ID NO: 49 |
| WT1 11 | AFTVHFSGQF | SEQ ID NO: 50 |
| WT1 12 | RWPSCQKKF | SEQ ID NO: 51 |
| WT1 13 | RVPGVAPTL | SEQ ID NO: 52 |
| WT1 14 | DFKDCERRF | SEQ ID NO: 53 |
| WT1 15 | RTPYSSDNL | SEQ ID NO: 54 |
| WT1 16 | TSEKPFSCR | SEQ ID NO: 55 |
| WT1 17 | FSRSDQLKR | SEQ ID NO: 56 |
| WT1 18 | LSHLQMHSR | SEQ ID NO: 57 |
| WT1 19 | YMFPNAPYL | SEQ ID NO: 58 |
| WT1 20 | CYTWNQMNL | SEQ ID NO: 59 |

Specifically, in the same manner as in <Example 4>, PBMCs isolated from the blood of ovarian cancer patients who gave written consent for the study were treated with 20 types of CTL peptides derived from the WT1 amino acid sequence of [Table 2] for 14 days, and re-stimulated for one day with the same peptide on the 14th day of culture. On the 15th day of culture, PBMCs in each tube were stained with anti-4-1BB-PE or anti-CD71-PE and anti-CD8-PE-Cy5. The proportion of CD71+CD8+ T cells and 4-1BB+CD8+T cells was analyzed through flow cytometry.

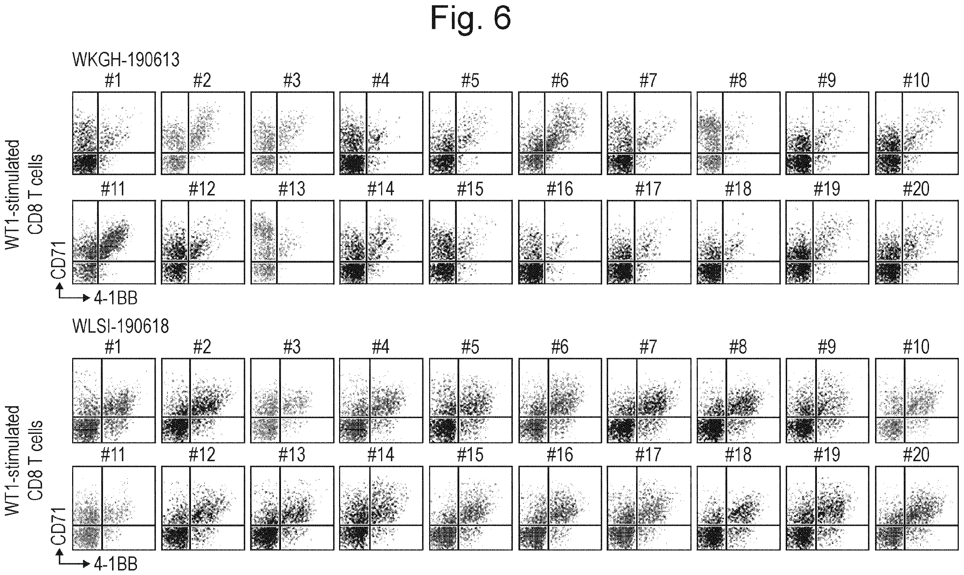

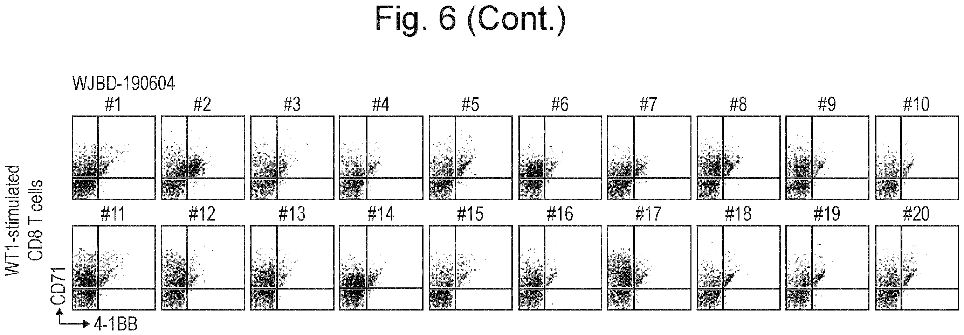

As a result, as shown in [Fig. 6], it was confirmed that 4-1BB⁺CD8 T cells were increased by various WT1 peptide stimuli.

In addition, as a result of flow cytometry analysis of the expression of CD71 and 4-1BB by gating only CD8 T cells on the 15th day of culture, 3 cases (red, yellow, and blue) were observed: 4-1BB⁺CD71⁺CD8 T cells with proportional expression of 4-1BB⁺CD71⁺CD8 T cells were mostly (red); a relatively large number of bystander-activated 4-1BB⁻CD71⁺CD8 T cells as well as 4-1BB⁺CD71⁺CD8 T cells were included (yellow); and the majority was bystander-activated 4-1BB⁻CD71⁺CD8 T cells (blue). Thus, it was confirmed that the proliferation of bystander-activated CD8 T cells and the proliferation of WT1 peptide-specific CD8 T cells occurred differently depending on the condition of the ovarian cancer patient and the type of WT1 peptide.

### [Example 6]

### Isolation of antigen-specific CD8 T cells using an automatic cell sorter

To isolate antigen-specific CD8+T cells with high purity under aseptic conditions, it was attempted to establish a separation process using an automatic cell separator.

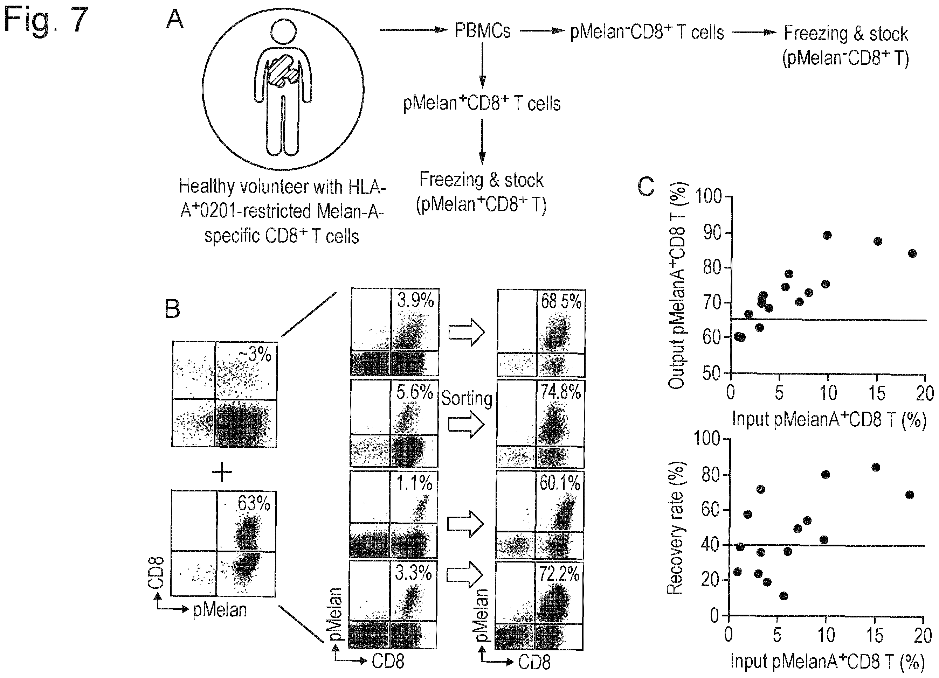

Specifically, in the same manner as in <Example 1>, blood was collected from HLA-A^{∗}0201-positive volunteers having Melan-A-specific CD8 T cells as autologous antigens to isolate PBMCs, and then melan-A CTL peptide was added to primary proliferation as peptide-specific CD8 T cells for 14 days. Primary cultured PBMCs were stained with PE-conjugated Melan-A peptide/MHC class I multimer (pMelan) and anti-CD8-PE-Cy5, and Melan-A-specific (pMelan+) and Melan-A-nonspecific (pMelan-) CD8+ T cells were isolated using a cell sorter (BD Bioscience, FACSAria). The isolated pMelan+CD8+ T cells and pMelan-CD8+ T cells were each mass-proliferated for 14 days by the rapid expansion method (FIG. 7A). The ratio of pMelan+CD8+ T cells was adjusted by mixing the final cultured pMelan+CD8+ T cells and pMelan-CD8+ T cells at various ratios, and pMelan+CD8+ T cells were separated by using Tyto (Miltenyi Biotec), which is capable of automatic cell separation in a sterile state, the purity recovery rate of pMelan+CD8+ T cells finally isolated according to the ratio of pMelan+CD8+ T cells was determined.

As a result, as shown in B of [Fig. 7], the final cultured pMelan+CD8+ T cells and pMelan-CD8+ T cells were confirmed at 63% and 3% levels, respectively.

In addition, pMelan+CD8+ T cells and pMelan-CD8+ T cells were mixed in various ratios so that the ratio of pMelan+CD8+ T cells was 3 to 20%, and then the purity of the isolated pMelan+CD8+ T cells was reconfirmed using Tyto. As a result, it was confirmed that the higher the ratio of pMelan+CD8+ T cells, the higher the ratio of pMelan+CD8+ T cells in the isolated cells. In addition, after isolating pMelan+CD8+ T cells under the condition of 5×10⁶ cells/ml, the number of pMelan+CD8+ T cells included in the sample before sorting was compared with the total number of finally isolated pMelan+CD8+ T cells for recovery rate. As a result of the analysis, it was found that when the ratio of pMelan+CD8+ T cells in the sample before sorting was 4% or more, 40% or more of the cells were stably collected after cell separation (FIG. 7C).

Overall, if >65% purity and >40% recovery rate were considered as the minimum criteria for target cell isolation, it was determined that the ratio of CD71+CD8+ T cells before cell isolation should be about 4% or more.

### [Industrial Applicability]

In case of isolating autologous cancer antigen-reactive CD8 T cells using the isolation method of the present invention, it is possible to provide significant anticancer effects by amplifying a large amount of CD8 T cells even with less blood since CD8 T cells responding to various cancer antigens in addition to CD8 T cells specific for autologous cancer antigen are also separated. Therefore, it can be effectively used for immunotherapy that induces temporary immunodeficiency for treatment such as adoptive cell therapy and thus it has great industrial applicability.

### SEQ ID Free Text

SEQ ID NO: 1 is the amino acid sequence of HLA-A0201/Melan-A 26-35 (Melan-A 26-35).
SEQ ID NO: 2 is an amino acid sequence of hTERT 1 as a CTL peptide derived from hTERT.
SEQ ID NO: 3 is an amino acid sequence of hTERT 2 as a CTL peptide derived from hTERT.
SEQ ID NO: 4 is an amino acid sequence of hTERT 3 as a CTL peptide derived from hTERT.
SEQ ID NO: 5 is an amino acid sequence of hTERT 4 as a CTL peptide derived from hTERT.
SEQ ID NO: 6 is an amino acid sequence of hTERT 5 as a CTL peptide derived from hTERT.
SEQ ID NO: 7 is an amino acid sequence of hTERT 6 as a CTL peptide derived from hTERT.
SEQ ID NO: 8 is an amino acid sequence of hTERT 7 as a CTL peptide derived from hTERT.
SEQ ID NO: 9 is an amino acid sequence of hTERT 8 as a CTL peptide derived from hTERT.
SEQ ID NO: 10 is an amino acid sequence of hTERT 9 as a CTL peptide derived from hTERT.
SEQ ID NO: 11 is an amino acid sequence of hTERT 10 as a CTL peptide derived from hTERT.
SEQ ID NO: 12 is an amino acid sequence of hTERT 11 as a CTL peptide derived from hTERT.
SEQ ID NO: 13 is an amino acid sequence of hTERT 12 as a CTL peptide derived from hTERT.
SEQ ID NO: 14 is an amino acid sequence of hTERT 13 as a CTL peptide derived from hTERT.
SEQ ID NO: 15 is an amino acid sequence of hTERT 14 as a CTL peptide derived from hTERT.
SEQ ID NO: 16 is an amino acid sequence of hTERT 15 as a CTL peptide derived from hTERT.
SEQ ID NO: 17 is an amino acid sequence of hTERT 16 as a CTL peptide derived from hTERT.
SEQ ID NO: 18 is an amino acid sequence of hTERT 17 as a CTL peptide derived from hTERT.
SEQ ID NO: 19 is the amino acid sequence of hTERT 18 as a CTL peptide derived from hTERT.
SEQ ID NO: 20 is an amino acid sequence of hTERT 19 as a CTL peptide derived from hTERT.
SEQ ID NO: 21 is an amino acid sequence of hTERT 20 as a CTL peptide derived from hTERT.
SEQ ID NO: 22 is an amino acid sequence of hTERT 21 as a CTL peptide derived from hTERT.
SEQ ID NO: 23 is an amino acid sequence of hTERT 22 as a CTL peptide derived from hTERT.
SEQ ID NO: 24 is an amino acid sequence of hTERT 23 as a CTL peptide derived from hTERT.
SEQ ID NO: 25 is an amino acid sequence of hTERT 24 as a CTL peptide derived from hTERT.
SEQ ID NO: 26 is an amino acid sequence of hTERT 25 as a CTL peptide derived from hTERT.
SEQ ID NO: 27 is an amino acid sequence of hTERT 26 as a CTL peptide derived from hTERT.
SEQ ID NO: 28 is an amino acid sequence of hTERT 27 as a CTL peptide derived from hTERT.
SEQ ID NO: 29 is an amino acid sequence of hTERT 28 as a CTL peptide derived from hTERT.
SEQ ID NO: 30 is an amino acid sequence of hTERT 29 as a CTL peptide derived from hTERT.
SEQ ID NO: 31 is an amino acid sequence of hTERT 30 as a CTL peptide derived from hTERT.
SEQ ID NO: 32 is an amino acid sequence of hTERT 31 as a CTL peptide derived from hTERT.
SEQ ID NO: 33 is an amino acid sequence of hTERT 32 as a CTL peptide derived from hTERT.
SEQ ID NO: 34 is an amino acid sequence of hTERT 33 as a CTL peptide derived from hTERT.
SEQ ID NO: 35 is an amino acid sequence of hTERT 34 as a CTL peptide derived from hTERT.
SEQ ID NO: 36 is an amino acid sequence of hTERT 35 as a CTL peptide derived from hTERT.
SEQ ID NO: 37 is an amino acid sequence of hTERT 36 as a CTL peptide derived from hTERT.
SEQ ID NO: 38 is an amino acid sequence of hTERT 37 as a CTL peptide derived from hTERT.
SEQ ID NO: 39 is an amino acid sequence of hTERT 38 as a CTL peptide derived from hTERT.
SEQ ID NO: 40 is the amino acid sequence of WT1 1 as a CTL peptide derived from WT1 cancer antigen.
SEQ ID NO: 41 is the amino acid sequence of WT1 2 as a CTL peptide derived from the WT1 cancer antigen.
SEQ ID NO: 42 is the amino acid sequence of WT1 3 as a CTL peptide derived from the WT1 cancer antigen.
SEQ ID NO: 43 is the amino acid sequence of WT1 4 as a CTL peptide derived from the WT1 cancer antigen.
SEQ ID NO: 44 is the amino acid sequence of WT1 5 as a CTL peptide derived from the WT1 cancer antigen.
SEQ ID NO: 45 is the amino acid sequence of WT1 6 as a CTL peptide derived from WT1 cancer antigen.
SEQ ID NO: 46 is the amino acid sequence of WT1 7 as a CTL peptide derived from WT1 cancer antigen.
SEQ ID NO: 47 is the amino acid sequence of WT1 8 as a CTL peptide derived from the WT1 cancer antigen.
SEQ ID NO: 48 is the amino acid sequence of WT1 9 as a CTL peptide derived from WT1 cancer antigen.
SEQ ID NO: 49 is the amino acid sequence of WT1 10 as a CTL peptide derived from WT1 cancer antigen.
SEQ ID NO: 50 is the amino acid sequence of WT1 11 as a CTL peptide derived from WT1 cancer antigen.
SEQ ID NO: 51 is the amino acid sequence of WT1 12 as a CTL peptide derived from WT1 cancer antigen.
SEQ ID NO: 52 is the amino acid sequence of WT1 13 as a CTL peptide derived from WT1 cancer antigen.
SEQ ID NO: 53 is the amino acid sequence of WT1 14 as a CTL peptide derived from WT1 cancer antigen.
SEQ ID NO: 54 is the amino acid sequence of WT1 15 as a CTL peptide derived from WT1 cancer antigen.
SEQ ID NO: 55 is the amino acid sequence of WT1 16 as a CTL peptide derived from WT1 cancer antigen.
SEQ ID NO: 56 is the amino acid sequence of WT1 17 as a CTL peptide derived from the WT1 cancer antigen.
SEQ ID NO: 57 is the amino acid sequence of WT1 18 as a CTL peptide derived from WT1 cancer antigen.
SEQ ID NO: 58 is the amino acid sequence of WT1 19 as a CTL peptide derived from the WT1 cancer antigen.
SEQ ID NO: 59 is the amino acid sequence of WT1 20 as a CTL peptide derived from WT1 cancer antigen.

## Claims

1. A method for isolating an autologous cancer antigen-specific CD8 T cell, comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a); and
d) isolating CD71+CD8+ T cells from the cultured PBMCs of step c).

2. The method for isolating an autologous cancer antigen-specific CD8 T cell of claim 1, wherein the cancer in step a) is at least one selected from the group consisting of gastric cancer, lung cancer, pancreatic cancer, melanoma, brain tumor, leukemia, ovarian cancer and sarcoma.

3. The method for isolating an autologous cancer antigen-specific CD8 T cell of claim 1, wherein the autologous cancer antigen is at least one selected from the group consisting of hTERT, WT-1, NY-ESO-1, and MAGE-3.

4. The method for isolating an autologous cancer antigen-specific CD8 T cell of claim 1, wherein the selection in step b) selects an autologous cancer antigen-derived peptide that highly expresses 4-1BB-expressing autologous cancer antigen-specific CD8 T cells.

5. The method for isolating an autologous cancer antigen-specific CD8 T cell of claim 1, wherein a blood volume of the cancer patient in step c) is 10 to 100 ml.

6. The method for isolating an autologous cancer antigen-specific CD8 T cell of claim 1, wherein the culturing in step c) is performed for 12 to 17 days.

7. An autologous cancer antigen-specific CD8 T cell isolated by the method of claim 1.

8. A pharmaceutical composition for preventing or treating cancer comprising the autologous cancer antigen-specific CD8 T cells of claim 7.

9. A method for improving or treating cancer comprising administering a composition comprising the autologous cancer antigen-specific CD8 T cells of claim 7 to a cancer patient.

10. A method for producing a composition for anticancer cell therapy comprising:
a) inducing proliferation of autologous cancer antigen-specific CD8 T cells after adding and culturing autologous cancer antigen-derived peptides to PBMCs derived from the blood of a cancer patient;
b) selecting an autologous cancer antigen-derived peptide having a high proliferation level of autologous cancer antigen-specific CD8 T cells in step a);
c) adding and culturing the autologous cancer antigen-derived peptide selected in step b) to PBMCs derived from the blood of the same cancer patient as in step a);
d) isolating CD71+CD8+ T cells from the cultured PBMC of step c); and
e) amplifying the CD71+CD8+ T cells isolated in step d).

11. The method for producing a composition for anticancer cell therapy of claim 10, wherein the separation of step d) is performed when the ratio of CD71+CD8+ T cells in PBMC is 1 to 25%.

12. The method for producing a composition for anticancer cell therapy of claim 10, wherein the amplification of step e) is performed for 12 to 17 days to amplify 10⁹ to 10¹¹ CD71+CD8+ T cells.

13. A composition for anticancer cell therapy prepared by the method of claim 10.
